# EUROPEAN PATENT SPECIFICATION

(11) **EP 2 155 251 B1**
(45) Date of publication and mention of the grant of the patent: **25.09.2013**
(21) Application number: 08826956.8
(22) Date of filing: 07.05.2008
(51) Int. Cl.: A61K 45/06, A61P 31/04

(54) **THERAPEUTIC COMPOSITIONS AND METHODS FOR TREATING GRAM-NEGATIVE BACTERIAL INFECTIONS**
THERAPEUTISCHE ZUSAMMENSETZUNGEN UND VERFAHREN ZUR BEHANDLUNG GRAM-NEGATIVER BAKTERIELLER INFEKTIONEN
COMPOSITIONS THÉRAPEUTIQUES ET MÉTHODES DE TRAITEMENT D'INFECTIONS BACTÉRIENNES GRAM-NÉGATIVES

(30) Priority: 08.05.2007 US 928398 P; 10.07.2007 US 959004 P
(43) Date of publication of application: 24.02.2010
(73) Proprietor: Rq Bioscience, Inc., Westborough, MA 01581 (US)
(72) Inventor: KAPPERS, Jeff, NL-1181 LC North Holland (NL); HEARN, Mark, Glenn, Abington, MA 02351 (US); QIU, Robert, Westborough, MA 01581 (US)
(74) Representative: Clegg, Richard Ian
(86) International application number: PCT/US2008/005877
(87) International publication number: WO 2009/020481

(56) References cited:
- US-A1- 2003 235 580
- US-A1- 2004 208 916
- US-A1- 2005 019 389
- SONG S-E ET AL: "Inhibitory effect of procyanidin oligomer from elm cortex on the matrix metalloproteinases and proteases of periodontopathogens" JOURNAL OF PERIODONTAL RESEARCH, BLACKWELL MUNKSGAARD, COPENHAGEN, vol. 38, no. 3, 1 June 2003 (2003-06-01), pages 282-289, XP002987940 ISSN: 0022-3484

## Description

### Background of the Invention

Nosocomial infections affect approximately two million, or 10%, of American hospital patients annually. Many of these infections are caused by opportunistic gram negative bacteria. The widespread use of antibiotics to treat bacterial infections has increased the prevalence of antibiotic-resistant strains, further exacerbating the problem.

Strains of Pseudomonas, such as *Pseudomonas aeruginosa,* are the cause of some of the most prevalent bacterial nosocomial infections. Of the two million nosocomial infections each year, 10% are caused by *P. aeruginosa.* The bacterium is the second most common cause of nosocomial pneumonia and the most common cause of intensive care unit (ICU) pneumonia. It is also the most common pathogen isolated from patients who have been hospitalized for more than one week.

Treatment of Pseudomonas infections is difficult in part because this bacterium tends to form biofilms that protect the organism against antibiotic treatment. *Pseudomonas aeruginosa* infections are known to occur in the urinary tract, respiratory system, heart, eye, ear, central nervous system, skin (e.g., in bums and wounds), and in the blood. Respiratory *P. aeruginosa* infections account for about 16% of all *Pseudomonas* infections.

Patients with cystic fibrosis are particularly vulnerable to respiratory *P. aeruginosa* infection. The infection typically starts with the bacteria binding to the epithelium in the patient's upper respiratory tract before migrating to the lung. The impairment of the ciliated epithelium in cystic fibrosis patients enables the infection to persist. Once in the lung, the bacteria produce alginate which impedes phagocytosis. Enzymes secreted by the bacteria cause lung tissue damage. Inflammation caused by the patient's immune response causes further lung damage. Patients with cystic fibrosis often develop pseudomonas infections as children and young adults and suffer recurrent attacks of pneumonia. The prevalence of such infections rises from about 30% in CF patients under the age of 10 to about 80% of patients between the ages of 18 to 44. It is believed that *P. aeruginosa* infection is now the primary cause of mortality in cystic fibrosis patients.

Other bacterial pathogens that have been more recently identified in CF patients are members of the Burkholderia cepacia complex ("BCC"). BCC is an important group of pathogens in immunocompromised hosts, also affecting patients with chronic granulomatous disease. BCC lung infections in certain patients with CF cause rapidly progressive, invasive and fatal disease. Moreover, BCC have a potential for patient-to-patient spreading.

Still other bacterial pathogens found in the lungs of CF patients include Staphylococcus aureus, methicillin-resistant Staphylococcus aureus (MRSA), Achromobacter xylosoxidans, Hemophilus influenza, and Stenotrophomonas maltophilia. S. aureus and H. influenza are most prevalent in patients in the first decade of life. S. maltophila infections afflict CF patients and other immunocompromised patients. Cystic Fibrosis Foundation, Patient Registry 2006 Annual Report, Bethesda, Maryland; Rajan S et al, Semin Respir Infect 2002, Mar 17(1):47-56; Denton M et al, Clin Microbiol Rev 1998, 11: 57-80; and Talmaciu I et al, Pediatr Pulmonol 2000, 30:10-15.

The biofilms formed in a *P. aeruginosa* infection present a serious hurdle in the treatment of bums and other wounds. Studies have revealed that approximately 10% of all burn patients contract a *P. aeruginosa* infection and over three-quarters of those patients do not survive. It is calculated that *P. aeruginosa* infection results in a 28% increase in mortality in burn victims. The topical antimicrobial agents used in burn care, such as sulphadiazine, cannot prevent colonization of burn wounds by *P. aeruginosa.*

In addition to nosocomial infections, *P. aeruginosa* is also a major cause of infections of the outer ear (otitis extrema, also known as "swimmer's ear) and middle ear (otitis media) in humans and dogs. Almost 1.25% of the human population and approximately 20% of the canine population is affected by otitis annually. In diabetic patients, chronic ear infections can occur, leading to paralysis. Chronic canine otitis leads to the presence of antibiotic resistant strains. Current treatment of severe or chronic otitis typically involves the use or aminoglycoside antibiotics, which have been associated with hearing damage.

Accordingly, there is a need for a safe, anti-bacterial therapy that can effectively be used to treat a patient suffering from or susceptible to gram-negative bacterial infection and which does not tend to cause antibiotic resistance.

### Summary of the Invention

The present invention relates to therapeutic compositions and said compositions for use in methods of treatment that contain or employ at least two of: an oligomeric proanthocyanidin; a protease inhibitor; and/or a lactoferrin. The compositions of this invention may be in various forms depending upon the disease or condition to be treated. The compositions of this invention are useful to treat a patient suffering from or susceptible to gram negative bacterial infections, including those associated with cystic fibrosis, ear infections and with wounds.

### Detailed Description of the Invention

The present invention addresses certain of the problems set forth above by providing compositions effective to treat a patient suffering from or susceptible to a gram negative bacterial infection.

### Pharmaceutical Compositions

In one embodiment, the invention provides a topical composition comprising: an oligomeric proanthocyanidin; a protease inhibitor; and a pharmaceutically and either a dermatologically or otically acceptable carrier.

In a more specific embodiment, the invention provides a topical composition comprising: an oligomeric proanthocyanidin; a protease inhibitor; a lactoferrin; and a pharmaceutically and either a dermatologically or otically acceptable carrier.

In another embodiment, the invention provides a topical composition comprising: a lactoferrin; a protease inhibitor; and a pharmaceutically and dermatologically acceptable carrier or an otically acceptable carrier.

In yet another embodiment, the invention provides an inhalable composition comprising a pharmaceutically acceptable carrier; and at least two components selected from: an oligomeric proanthocyanidin; a protease inhibitor; and a lactoferrin.

In another embodiment the invention provides an inhalable composition comprising: an oligomeric proanthocyanidin; and a lactoferrin.

In still another embodiment, the invention provides an oral or buccal composition comprising a lactoferrin; at least one agent selected from an oligomeric proanthocyanidin and a protease inhibitor; and a pharmaceutically acceptable carrier.

In yet another embodiment the invention provides an oral or buccal composition comprising a lactoferrin; an oligomeric proanthocyanidin; and a pharmaceutically acceptable carrier.

In still another embodiment the invention provides an oral or buccal composition comprising a lactoferrin; a protease inhibitor; and a pharmaceutically acceptable carrier.

Proanthocyanidins are polymers of flavanols. Flavanols have the general formula: 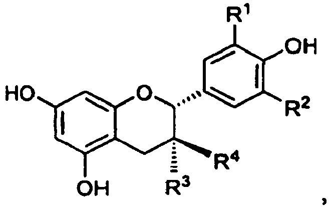 wherein each R is defined in the table below:

| Proanthocyanidin Subclass | Flavanol monomer | R¹ | R² | R³ | R⁴ |
|---|---|---|---|---|---|
| Procyanidin | Catechin | OH | H | H | OH |
| Procyanidin | Epicatechin | OH | H | OH | H |
| Prodelphinidin | Gallocatechin | OH | OH | H | OH |
| Prodelphinidin | Epigallocatechin | OH | OH | OH | H |
| Propelargonidin | Afzelechin | H | H | H | OH |

Epicatechin can also be optionally esterified at the R³ and/or R⁴ by gallic acid in the procyanidins of this invention. Thus, the term "epicatechin" as used herein, includes the 3-gallate and the 3,3'-di-gallate esters thereof unless otherwise specified. Similarly, the term "procyanidin", as used herein, includes molecules comprising the 3-gallate and the 3,3'-di-gallate esters of epicatechin, unless otherwise specified.

Oligomeric proanthocyanidins utilized in this invention may consist of 2, 3, 4, 5, 6, 7, 8, or 9 flavonol monomers linked to one another, or any combinations thereof. Individual monomers may be linked to one another via one bond ("B" type; C4→C6, or C4→C8) or two bonds ("A" type; both C4→C8 and C2→C7). In certain embodiments, the linkages between monomers in the oligomeric proanthocyanidins present in the compositions of this invention are at least 50%, at least 60%, at least 70%, at least 80%, at least 90%, or at least 95% of the B type.

In other embodiments, the oligomeric proanthocyanidins present in the compositions of this invention are at least 50%, at least 60%, at least 70%, at least 80%, at least 90%, or at least 95% composed of flavonol monomers of the same proanthocyanidin subclass.

In other embodiments, the oligomeric proanthocyanidins present in the compositions of this invention are at least 50%, at least 60%, at least 70%, at least 80%, at least 90%, or at least 95% composed of catechin and epicatechin monomers.

In another embodiment, the oligomeric proanthocyanidins present in the compositions of this invention are at least 50%, at least 60%, at least 70%, at least 80%, at least 90%, or at least 95% composed of catechin and epicatechin monomers; and are at least 50%, at least 60%, at least 70%, at least 80%, at least 90%, or at least 95% B type.

In still another embodiment, at least 50%, at least 60%, at least 70%, at least 80%, at least 90%, or at least 95% by weight of the oligomeric proanthocyanidins present in the compositions of this invention are dimeric, trimeric and tetrameric procyanidins.

The proanthocyanidins utilized in the present inventions may be chemically synthesized. The synthesis of various proanthocyanidins is known in the art and is disclosed in, for example, WO1999/109319; WO 2000/063201; Steynberg, P.J. et al., Tetrahedron, 1998, 54(28): 8153; Tueckmantel, W. et al., J Am Chem Soc 1999, 121(51): 12073; Saito, A. et al., Bioorg Med Chem, 2004, 12(18):4783-90; Kozikowski, A.P., J Org, Chem, 2003, 68(5):1641-58, the disclosures of which are herein incorporated by reference in their entirety.

Alternatively, the proanthocyanidins utilized in the present inventions may be extracted from a natural source. Proanthocyanidins and particularly procyanidins are found in fruit, vegetables, nuts, seeds, flowers and bark (see USDA Database for the Proanthocyanidin Content of Selected Foods, 2004, http://www.nal.usda.gov/fnic/foodcomp/Data/PA/PA.pdf). Particularly rich sources of procyanidins are maritime pine bark and grape seeds. The extraction of proanthocyanidins from various natural sources is known in the art (see, for example, United States Patent 5,912,363; and WO 2006/112496), the disclosure of which are herein incorporated by reference in their entirety.

The oligomeric proanthocyanidins utilized in the present invention may be present as an extract from a natural source; as individual or combinations of oligomeric proanthocyanidins isolated from such an extract; as individual or combinations of chemically synthesized oligomeric proanthocyanidins; or as a combination of any of the foregoing.

In one embodiment, the oligomeric proanthocyanidin component utilized in the present invention is a grape seed extract. Grape seed extract is commercially available. In another embodiment, the oligomeric proanthocyanidin component utilized in the present invention is a grape seed proanthocyanidin extract known under the Acti Vin® brand name and available from InterHealth Nutraceuticals, Inc, Benicia, California.

Eight different procyanidin dimers are known to exist in grape seed extracts and they are termed B1, B2, B3, B4, B5, B6, B7 and B8:

| | |
|---|---|
| 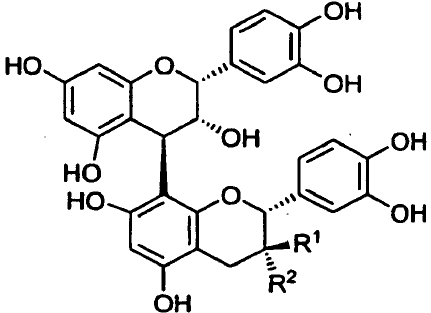 | Procyanidin B₁: R¹=OH; R²=H |
| | Procyanidin B₂: R¹=H; R²=OH |
| 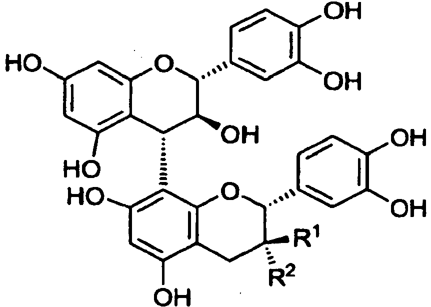 | Procyanidin B₃: R¹=OH; R²=H |
| | Procyanidin B₄: R¹=H; R²=OH |
| 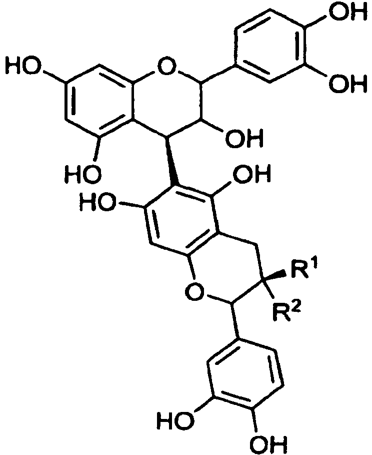 | Procyanidin B₅: R¹=H; R²=OH |
| | Procyanidin B₇: R¹=OH; R²=H |
| 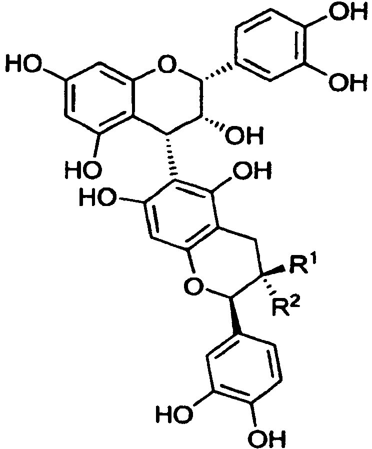 | Procyanidin B₆: R¹=OH; R²=H |
| | Procyanidin B₈: R¹=H; R²=OH |

In addition, six different procyanidin trimers and a wide variety of tetramers and higher oligomeric procyanidins are found in grape seed extract. Grape seed extracts, including ActiVin® may be further purified by well-known separation methods, such as HPLC, to remove or enrich for certain procyanidin species before use in the present invention.

The term "protease inhibitor" as used herein in reference to a component of the compositions of this invention or to a component used in the methods of this invention, refers to a molecule distinct from an oligomeric proanthocyanidin. Oligomeric proanthocyanidins have been disclosed to have certain indirect protease inhibitory activity (see, e.g., US Patent Publication 20040234480), but are not intended to be encompassed within the term "protease inhibitor" as used in this application.

In one embodiment, the compositions of this invention comprise a protease inhibitor that inhibits a protease secreted by gram negative bacteria. In another embodiment, the protease inhibitor is a compound that inhibits a protease secreted by Pseudomonas aeruginosa. In a specific embodiment, the protease inhibitor is a compound that inhibits one or more of bacterial LasA elastase, LasB elastase, alkaline protease, neutrophile elastase, cathepsins, macrophage elastase, acid esterases, collagenases, tryptases, chymases, kinins, kallikreins, tumor necrosis factors, chymotrypsins, stromelysins, and matrix metalloproteases.

In some embodiments, the protease inhibitor is selected from an aspartic acid protease inhibitor, a serine protease inhibitor, a cysteine protease inhibitor, and a metalloprotease inhibitor.

In other embodiments, the protease inhibitor is a multispecific protease inhibitor (i.e., one that targets multiple proteases). In a specific embodiment, the multispecific protease inhibitor is selected from α1-antiprotease, α2-macroglobulin and secretory leucocyte protease inhibitor.

In other embodiments, the protease inhibitor is an oligospecific or specific protease inhibitor. In certain embodiments, the oligospecific or specific protease inhibitor is selected from β1-antigellagenase, α2-antiplasmin, serine amyloid A protein, α1-antichymotrypsin, cystatin C, inter-α-trypsin inhibitor, elafin, elastinal, aprotinin, phenylmethyl sulfonyl fluoride, E-64, leupeptin, TIMP-1, TIMP-2, and 1,10-phenanthroline.

In still other embodiments, more than one protease inhibitor is present in the compositions of this invention. In certain embodiments, at least two different multispecific protease inhibitors are present. In other embodiments, at least one multi specific protease inhibitor and at least one oligospecific or specific protease inhibitor is present in the compositions of this invention.

The protease inhibitor component of the compositions of this invention may be isolated from natural sources or produced by recombinant methods.

Lactoferrins useful in the present invention include native lactoferrin isolated from mammals, recombinantly produced lactoferrin, N-terminal variants of lactoferrin in which at least the N-terminal glycine is varied, lactoferrin containing point mutations, such as the R210K mutation in human lactoferrin, and active fragments of lactoferrin. Native lactoferrin can be obtained by purification from mammalian milk or colostrum or from other natural sources. See, for example, United States Patent Publication 20060093592, the disclosure of which is herein incorporated by reference in its entirety. Recombinant lactoferrin can be made by recombinant expression or direct production in genetically altered animals, plants, fungi, bacteria, or other prokaryotic or eukaryotic species, or through chemical synthesis. Active fragments of lactoferrin can be made recombinantly, synthetically or by proteolytic digestion of full-length native or recombinant lactoferrin.

In one embodiment, the lactoferrin is a recombinantly produced human lactoferrin, also known as talactoferrin alpha, such as that disclosed in WO1993/022348, the disclosure of which is herein incorporated by reference.

In another embodiment, the lactoferrin is a fragment selected from human lactoferrin fragments described in WO2001/034641; bovine lactoferrin fragments described in JP 2004002471; recombinantly produced truncated lactoferrin described in United States Patent 6,333,311; human lactoferricin (residues 1-47 of human lactoferrin); bovine lactoferricin (residues 17 to 41 of bovine lactoferrin); other pepsin digestion-produced fragments described in Vorland, L.H., APMIS, 1999, 107:971-81; peptides based upon portions of human or bovine lactoferricins described in Lejon, T et al., J Pept Sci., 2004 Jun, 10(6):329-35, Nibbering PH et al., Infect Immun., 2001, Mar, 69(3):1469-76, Haversen LA et al., Infect Immun., 2000 Oct, 68(10):5816-23; human lactoferrin fragment comprising the complete N-lobe (see, for example, Tanaka, T. et al., Biochem Cell Biol, 2003, 81:349-54); and a human lactoferrin fragment comprising the complete C-lobe (see, for example, Kim, W-S et al., Bioscience, Biotechnology, and Biochemistry, 2006, 70:2641-2645).

In another embodiment, the lactoferrin is a lactoferrin fragment selected from human lactoferrin (1-11), human lactoferrin (1-47), bovine lactoferrin (1-51), bovine lactoferrin (17-41), human lactoferrin (1-333), human lactoferrin (345-692) and human lactoferrin (21-31).

In another embodiment, the lactoferrin is an N-terminal lactoferrin variant, such as described in WO2004/103285.

In still another embodiment, the lactoferrin is bovine lactoferrin. Bovine lactoferrin includes bovine apolactoferrin, such as is sold under the name Bioferrin® (Glanbia Nutritionals, Inc., Monroe WI, USA).

The compositions of the present invention may conveniently be presented in unit dosage form and may be prepared by any methods well known in the art of pharmacy. The amount of active ingredients which can be combined with a carrier material to produce a single dosage form will vary depending upon the host being treated, the particular mode of administration. The amount of active ingredients that can be combined with a carrier material to produce a single dosage form will generally be that amount of each compound which produces a therapeutic effect. Generally, out of one hundred percent, the total amount will range from about 1 percent to about ninety-nine percent of active ingredients, preferably from about 5 percent to about 70 percent, most preferably from about 10 percent to about 30 percent.

Methods of preparing these compositions include the step of bringing into association the active compounds contained therein with the carrier and, optionally, one or more accessory ingredients. In general, the formulations are prepared by uniformly and intimately bringing into association a compound of the present invention with liquid carriers, or finely divided solid carriers, or both, and then, in the case of certain solid oral or buccal formulation, shaping the product.

The topical compositions of the present invention further comprise a pharmaceutically and dermatologically acceptable carrier or an otically acceptable carrier. The carrier(s) must be "acceptable" in the sense of not being deleterious to the recipient thereof in amounts typically used in medicaments. Such carriers, in the case of dermatologically acceptable carriers, are preferably compatible with skin, nails, mucous membranes, tissues and/or hair, and can include any conventionally used dermatological carrier meeting these requirements. In the case of otically acceptable carriers, the carrier is preferably compatible with all parts of the ear. Such carriers can be readily selected by one of ordinary skill in the art. Carriers for topical administration of the compounds of this invention include, but are not limited to, mineral oil, liquid petroleum, white petroleum, propylene glycol, polyoxyethylene and/or polyoxypropylene compounds, emulsifying wax, sorbitan monostearate, polysorbate 60, cetyl esters wax, cetearyl alcohol, 2-octyldodecanol, benzyl alcohol, and water. In formulating skin ointments, the active components of the instant invention may be formulated in an oleaginous hydrocarbon base, an anhydrous absorption base, a water-in-oil absorption base, an oil-in-water water-removable base and/or a water-soluble base. In formulating otic compositions, the active components of the present invention may be formulation in an aqueous polymeric suspension including such carriers as dextrans, polyethylene glycols, polyvinylpyrrolidone, polysaccharide gels, Gelrite®, cellulosic polymers like hydroxypropyl methylcellulose, and carboxy-containing polymers such as polymers or copolymers of acrylic acid, as well as other polymeric demulcents.

The topical compositions according to the present invention may be in any form suitable for topical application, including aqueous, aqueous-alcoholic or oily solutions, lotion or serum dispersions, aqueous, anhydrous or oily gels, emulsions obtained by dispersion of a fatty phase in an aqueous phase (O/W or oil in water) or, conversely, (W/O or water in oil), microemulsions or alternatively microcapsules, microparticles or lipid vesicle dispersions of ionic and/or nonionic type, creams, lotions, gels, essences, milks, suspensions, or patches.

Topical compositions of the present invention may also contain adjuvants such as hydrophilic or lipophilic gelling agents, hydrophilic or lipophilic active agents, preserving agents, antioxidants other than oligomeric proanthocyanidins, solvents, fragrances, fillers, sunscreens, odor-absorbers and dyestuffs. The amounts of these various adjuvants are those conventionally used in the fields considered and, for example, are from about 0.01% to about 20% of the total weight of the composition. Depending on their nature, these adjuvants may be introduced into the fatty phase, into the aqueous phase and/or into the lipid vesicles.

In one embodiment, the topical compositions of this invention additionally comprise one or more components used to treat topical bums. Such components include, but are not limited to, a propylene glycol hydrogel (e.g., SOLUGEL® (Johnson & Johnson)); a combination of a glycol, a cellulose derivative and a water soluble aluminum salt, such as described in US 6,958,159; an antiseptic; an antibiotic other than a lactoferrin or an oligomeric proanthocyanidin; and a corticosteroid.

In another embodiment, the topical compositions of this invention additionally comprise one or more components used for otitis. Such components include antibiotics other than a lactoferrin or an oligomeric proanthocyanidin, such as an azalide antibiotic disclosed in United States Patent Publication 20060046970; steroidal or non-steroidal antiinflammatory agent; antivirals; antifungals; anesthetics; and anti-allergic agents.

In another embodiment, the composition is an inhalable composition. An "inhalable composition" refers to certain pharmaceutical compositions of the present invention that are formulated for direct delivery to the respiratory tract during routine or assisted respiration (e.g., by intratracheobronchial, pulmonary, and/or nasal administration), including, but not limited to, atomized, nebulized, dry powder and/or aerosolized formulations.

The inhalable compositions of this invention comprise a pharmaceutically acceptable carrier. The term "pharmaceutically acceptable carrier" includes any and all solvents, additives, excipients, dispersion media, solubilizing agents, coatings, preservatives, isotonic and absorption delaying agents, surfactants, propellants and the like that are physiologically compatible.

A surfactant can be added to an inhalable pharmaceutical composition of the present invention to lower the surface and interfacial tension between the medicaments and the propellant. Where the medicaments, propellant and excipient are to form a suspension, a surfactant may or may not be required. Where the medicaments, propellant and excipient are to form a solution, a surfactant may or may not be necessary, depending in part, on the solubility of the particular medicament and excipient. The surfactant may be any suitable, non-toxic compound which is non-reactive with the medicament and which substantially reduces the surface tension between the medicament, the excipient and the propellant and/or acts as a valve lubricant.

Examples of suitable excipients include, but are not limited to: lactose, starch, propylene glycol diesters of medium chain fatty acids; triglyceride esters of medium chain fatty acids, short chains, or long chains, or any combination thereof; perfluorodimethylcyclobutane; perfluorocyclobutane; polyethylene glycol; menthol; lauroglycol; diethylene glycol monoethylether; polyglycolized glycerides of medium chain fatty acids; alcohols; eucalyptus oil; short chain fatty acids; and combinations thereof.

More specific excipients that may be utilized in the inhalable compositions of this invention are: propylene glycol diesters of medium chain fatty acids available under the trade name Miglyol 840 (from Huls America, Inc. Piscataway, N.J.); triglyceride esters of medium chain fatty adds available under the trade name Miglyol 812 (from Huls); perfluorodimethylcyclobutane available under the trade name Vertrel 245 (from E. I. DuPont de Nemours and Co., Inc. Wilmington, Del.); perfluorocyclobutane available under the trade name octafluorocyclobutane (from PCR, Gainesville, Fla.); polyethylene glycol available under the trade name PEG 400 (from BASF Parsippany, N.J.); menthol (from Pluess-Stauffer International, Stanford, Conn.); propylene glycol monolaurate available under the trade name lauroglycol (from Gattefosse, Elmsford, N.Y.); diethylene glycol monoethylether available under the trade name Transcutol (from Gattefosse); polyglycolized glyceride of medium chain fatty acids available under the trade name Labrafac Hydro WL 1219 (from Gattefosse); alcohols, such as ethanol, methanol and isopropanol; eucalyptus oil (available from Pluses-Stauffer International); and mixtures thereof.

Examples of suitable propellants include, but are not limited to: dichlorodifluoromethane, trichlorofluoromethane, dichloro-tetrafluoroethane and carbon dioxide.

Examples of suitable surfactants include, but are not limited to: oleic acid; sorbitan trioleate; cetyl pyridinium chloride; soya lecithin; polyoxyethylene(20) sorbitan monolaurate; polyoxyethylene (10) stearyl ether; polyoxyethylene (2) oleyl ether; polyoxypropylene-polyoxyethylene ethylene diamine block copolymers; polyoxyethylene(20) sorbitan monostearate; polyoxyethylene(20) sorbitan monooleate; polyoxypropylene-polyoxyethylene block copolymers; castor oil ethoxylate; and combinations thereof.

More specific surfactants that may be utilized in the inhalable compositions of this invention are: oleic acid available under the trade name oleic acid NF6321 (from Henkel Corp. Emery Group, Cincinnati, Ohio); cetylpyridinium chloride (from Arrow Chemical, Inc. Westwood, N.J.); soya lecithin available under the trade name Epikuron 200 (from Lucas Meyer Decatur, Ill.); polyoxyethylene(20) sorbitan monolaurate available under the trade name Tween 20 (from ICI Specialty Chemicals, Wilmington, Del.); polyoxyethylene(20) sorbitan monostearate available under the trade name Tween 60 (from ICI); polyoxyethylene(20) sorbitan monooleate available under the trade name Tween 80 (from ICI); polyoxyethylene (10) stearyl ether available under the trade name Brij 76 (from ICI); polyoxyethylene (2) oleyl ether available under the trade name Brij 92 (frown ICI); Polyoxyethylene-polyoxypropylene ethylenediamine block copolymer available under the tradename Tetronic 150 RI (from BASF); polyoxypropylene-polyoxyethylene block copolymers available under the trade names Pluronic L-92, Pluronic L-121 end Pluronic F 68 (from BASF); castor oil ethoxylate available under the trade name Alkasurf CO-40 (from Rhone-Poulenc Mississauga Ontario, Canada) ; and mixtures thereof. These surfactants may be utilized either as the free base, as a salt, or as a clathrate (such as P-11 or hexane clathrates), depending upon the stability and solubility of the active compounds in the specific pharmaceutical composition.

The inhalable compositions of this invention, when in liquid form are typically aerosolized and delivered via nebulization. Nebulizers for delivering an aerosolized solution include the AERx® (Aradigm), the Ultravent® (Mallinckrodt), and the Acorn II® (Marquest Medical Products).

In various embodiments, the pharmaceutical compositions of the present invention are formulated for administration by dry powder inhalation and can comprise one or more additional chemicals and entities which facilitate the administration of the active ingredient.

Pharmaceutical excipients and additives useful in the dry powder compositions of this invention include but are not limited to proteins, peptides, amino acids, lipids, and carbohydrates (e.g., sugars, including monosaccharides, di-, tri-, tetra-, and oligosaccharides; derivatized sugars such as alditols, aldonic acids, esterified sugars and the like; and polysaccharides or sugar polymers such as Ficolls), which may be present singly or in combination. Preferred are excipients that are soluble in water (e.g., sugars, peptides, amino acids, and salts), alcohol (such as pectin, lecithin, and povidone) or acetone (e.g., citric acid, PLGA). Also preferred are excipients having a glass transition temperature (Tg), above about 35°C, preferably above about 45°C, more preferably above about 55°C. Illustrative excipients suitable for use in the dry powder compositions of the invention include those disclosed in International Patent Publication Nos. WO98/16205, WO 96/32096, United States Patent 7,112,341.

Delivery of dry powder compositions is typically achieved with an inhaler. A wide variety of dry-powder inhalers can be used to administer various embodiments of dry powder formulations of the pharmaceutical compositions of the present invention, such as those described in United States Patent Nos. 5,458,135; 5,740,794; 5,785,049; 4,013,075; 5,522,385; 4,668,218; 4,667,668; 4,805,811; and 5,388,572; and in European Patent Publication Nos. EP 129985; EP472598; and EP 467172. Examples of commercially available dry-powder inhalers include, but are not limited to, Diskus®, Diskhaler®, and Rotahaler® brand inhalers (GlaxoSmithKline, Inc.), the Turbuhaler® brand inhaler (AstraZeneca), the HandiHaler® brande inhaler (Boehringer Ingelheim Pharma KG), and the Aerolizer brand inhaler® (Novartis).

Alternatively, the dry powder compositions of this invention can be delivered using a pressurized, metered dose inhaler (MDI), e.g., the Ventolin® metered dose inhaler, containing a solution or suspension of a composition of this invention in a pharmaceutically inert liquid propellant, e.g., a chlorofluorocarbon or fluorocarbon, as described in United States Patent Nos. 5,320,094, and 5,672,581.

In one embodiment, the majority of aerosolized powder particles have an aerodynamic diameter of less than 3.3µm, typically determined in an Andersen cascade impactor. These sized particles have the greatest potential for deep lung penetration in a patient. The term "patient", as used herein, means a mammal, preferably a human or a domesticated animal, such as a dog.

In one embodiment, the inhalable compositions of the present invention additionally comprise an additional therapeutic agent useful in the treatment of lung infections and/or cystic fibrosis. These additional therapeutic agents include mucus dissolvers, such as dornase alfa (Pulmozyme®); and antibiotics other than a lactoferrin or an oligomeric proanthocyanidin, such as colstin, tobramycin, azithromycin and ciprofloxacin.

Compositions of the invention suitable for oral or buccal administration may be in the form of capsules, cachets, pills, tablets, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouthwashes and the like, each containing a predetermined amount of a compound of the present invention as an active ingredient. Compositions or compounds may also be administered as a bolus, electuary or paste.

To prepare solid dosage forms for oral administration (capsules, tablets, pills, dragees, powders, granules and the like), the active ingredient is mixed with one or more pharmaceutically acceptable carriers, such as sodium citrate or dicalcium phosphate, and/or any of the following: (1) fillers or extenders, such as starches, lactose, sucrose, glucose, mannitol, and/or silicic acid; (2) binders, such as, for example, carboxymethylcellulose, alginates, gelatin, polyvinyl pyrrolidone, sucrose and/or acacia; (3) humectants, such as glycerol; (4) disintegrating agents, such as agar-agar, calcium carbonate, potato or tapioca starch, alginic acid, certain silicates, and sodium carbonate; (5) solution retarding agents, such as paraffin; (6) absorption accelerators, such as quaternary ammonium compounds; (7) wetting agents, such as, for example, cetyl alcohol and glycerol monostearate; (8) absorbents, such as kaolin and bentonite clay; (9) lubricants, such a talc, calcium stearate, magnesium stearate, solid polyethylene glycols, sodium lauryl sulfate, and mixtures thereof; and (10) coloring agents. In the case of capsules, tablets and pills, the pharmaceutical compositions may also comprise buffering agents. Solid compositions of a similar type may also be employed as fillers in soft and hard-filled gelatin capsules using such excipients as lactose or milk sugars, as well as high molecular weight polyethylene glycols and the like.

A tablet may be made by compression or molding, optionally with one or more accessory ingredients. Compressed tablets may be prepared using binder (for example, gelatin or hydroxypropylmethyl cellulose), lubricant, inert diluent, preservative, disintegrant (for example, sodium starch glycolate or cross-linked sodium carboxymethyl cellulose), surface-active or dispersing agent. Molded tablets may be made by molding in a suitable machine a mixture of the powdered compound moistened with an inert liquid diluent.

The tablets, and other solid oral or buccal dosage forms of the pharmaceutical compositions, such as dragees, capsules, pills and granules, may optionally be scored or prepared with coatings and shells, such as enteric coatings and other coatings well known in the pharmaceutical-formulating art. They may also be formulated so as to provide slow or controlled release of the active ingredient therein using, for example, hydroxypropylmethyl cellulose in varying proportions to provide the desired release profile, other polymer matrices, liposomes and/or microspheres. They may be sterilized by, for example, filtration through a bacteria-retaining filter, or by incorporating sterilizing agents in the form of sterile solid compositions that can be dissolved in sterile water, or some other sterile injectable medium immediately before use. These compositions may also optionally contain opacifying agents and may be of a composition that they release the active ingredient(s) only, or preferentially, in a certain portion of the gastrointestinal tract, optionally, in a delayed manner. Examples of embedding compositions that can be used include polymeric substances and waxes. The active ingredient can also be in micro-encapsulated form, if appropriate, with one or more of the above-described excipients.

Liquid dosage forms useful for oral administration include pharmaceutically acceptable emulsions, microemulsions, solutions, suspensions, syrups and elixirs. In addition to the active ingredient, the liquid dosage forms may contain inert diluents commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof.

Besides inert diluents, the oral compositions can also include adjuvants such as wetting agents, emulsifying and suspending agents, sweetening, flavoring, coloring, perfuming and preservative agents.

Suspensions, in addition to the active compounds, may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

In the pharmaceutical compositions of the invention, the compound of the present invention is present in an effective amount. As used herein, the term "effective amount" refers to an amount which, when administered in a proper dosing regimen, is sufficient to reduce or ameliorate the severity, duration or progression of the disorder being treated, prevent the advancement of the disorder being treated, cause the regression of the disorder being treated, or enhance or improve the prophylactic or therapeutic effect(s) of another therapy.

The interrelationship of dosages for animals and humans (based on milligrams per meter squared of body surface) is described in Freireich et al., Cancer Chemother. Rep. 1966, 50: 219. Body surface area may be approximately determined from height and weight of the patient. *See, e*.*g*., Scientific Tables, Geigy Pharmaceuticals, Ardsley, N.Y., (1970) 537.

Oligomeric proanthocyanidins and lactoferrins are well tolerated by patients and exhibit little or no toxicity. Thus, effective amounts of a lactoferrin range from about 10 mg/day to about 10 g/day. Effective amounts of an oligomeric proanthocyanidin range from about 500 µg/day to about 500 mg/day.

Effective amounts of a protease inhibitor in the compositions of this invention will vary with the type and nature of protease inhibitor. Such dosages are well-known in the art. For example, alpha-1 antitrypsin is typically administered at a dosage of about 60 mg/kg body weight.

### Inhalable Composition Delivery Systems

In another embodiment, the present invention provides a delivery device for an inhalable composition of this invention comprising: a dispenser portion; a sealed container comprising a composition of the present invention and a propellant; and a compressive portion that opens the sealed container.

In one embodiment, the delivery device is adapted to deliver the inhalable composition of this invention as particles or droplets.

In an alternate embodiment, the delivery device is adapted to deliver the inhalable composition as a dry powder.

The choice of dispenser portion of the apparatus of this invention is dependent upon the nature of the inhalable composition. For liquid or suspension inhalable compositions, the dispenser is a nebulizer, such as the AERx® (paradigm), the Ultravent® (Mallinkrodt), and the Acorn II® (Marquest Medical Products). For dry powder inhalable compositions, the dispenser is either a dry powder inhaler or a metered dose inhaler. Examples of dry powder inhalers useful in this invention include Spiros® inhaler (Dura Pharmaceuticals), the Spinhaler® (Fisons), Diskus®, Diskhaler®, and Rotahaler® brand inhalers (GlaxoSmithKline, Inc.), the Turbuhaler® brand inhaler (AstraZeneca), the HandiHaler® brand inhaler (Boehringer Ingelheim Pharma KG), the Aerolizer brand inhaler® (Novartis), and the CR-60 high flow compressor (Romedic BV).

The dispenser typically comprises a holding part and a nozzle. The holding part is arranged to retain the sealed container which comprises the inhalable composition of this invention and a propellant. The nozzle is arranged to guide the expelled medicament into the nose or mouth (as required) of the patient during use. The holding part preferably includes retaining means to retain the sealed flexible container in place. For example, the holding part may include one or more recesses or the like for receiving and retaining an edge portion of the sealed container.

The container may be composed of flexible or rigid material. In one embodiment, the container is made of a rigid material, such as metal, and is designed to hold multiple doses of an inhalable composition of this invention.

The seal of the sealed container must be openable so as to release a dose of the composition contained therein. The sealed flexible container comprises at least one unit dose of the inhalable composition, which is specifically adapted for use with the particular dispenser. If designed to contain only a single dose, the sealed container may be pierceable or otherwise frangible so as to release its entire content. If the container is designed to hold multiple doses, the seal must be both openable and re-closeable after delivering a dose of the composition. This may be achieved, for example, by means of a stopper or diaphragm. If the container comprises multiple unit doses of the composition, it is further adapted to deliver one unit dose at a time.

The compressive portion of the delivery device interlocks or otherwise is in physical communication with the dispenser such that when the compressive part is actuated, the sealed container opens and the composition released from the container enters the delivery device.

### Wound Dressings

In another embodiment, the invention provides a wound dressing comprising a pre-applied composition, wherein the pre-applied composition comprises: a pharmaceutically and dermatologically acceptable carrier; and at least two components selected from: a) an oligomeric proanthocyanidin; b) a protease inhibitor; and c) a lactoferrin.

Protease inhibitors that are useful in the wound dressings and wound treatment methods of the present invention are the same as those set forth above as useful in the compositions of the present invention.

The pre-applied composition may be physically embedded in the wound dressing, cross-linked or otherwise chemically attached to the wound-dressing, exist as a layer on a surface of the wound dressing to be contacted with a wound, or be associated with the wound dressing in any other manner that would allow the composition to come into contact with the wound after the dressing is applied thereto.

In some embodiments, the wound dressing further comprises a hydrogel or hydrogel-forming composition to maintain a moist environment, as described, for example, in US Patent 5,527,271 and US Patent Publication 20050214376.

In some embodiments, the wound dressing further comprises, either as part of the same pre-applied composition or separate from that composition, one or more agents used to treat topical wounds. Such components include, but are not limited to, a propylene glycol hydrogel (e.g., SOLUGEL® (Johnson & Johnson)); a combination of a glycol, a cellulose derivative and a water soluble aluminum salt, such as described in US 6,958,159; an antiseptic; an antibiotic other than a lactoferrin or an oligomeric proanthocyanidin; and a corticosteroid.

In certain embodiments, the portion of the wound dressing comprising the pre-applied composition and/or any additional components used to treat topical bums is covered with a removable protective coating, such as a plastic strip. The coating protects the composition and any additional components from the environment and is removed prior to contacting the dressing with the wound.

### Methods of Treatment

The disclosure is useful in a method of treating a patient susceptible to or suffering from a gram negative bacterial infection comprising the step of co-administering to a patient in need thereof at least two of: a) an effective amount of a lactoferrin; b) an effective amount of an oligomeric proanthocyanidin; and c) an effective amount of a protease inhibitor.

The term "co-administer" as used herein means that the recited agents may be administered together as part of a single dosage form or as separate, multiple dosage forms. The separate dosage forms utilized in a co-administration need not be of the same type (i.e., oral, buccal, topical or inhalable). Similarly, in certain embodiments for treating bums and wounds, one dosage form may be applied to a wound dressing, while another used in the co-administration may be applied directly to the wound. In addition to simultaneous adminstration of separate dosage forms, the term "co-administer" also includes treatement wherein one recited agent is administered prior to, consecutively with, or following the administration of another recited agent. The administration of a composition of this invention, comprising multiple agents to a patient does not preclude the separate administration of one of those agents, or any agent at another time during a course of treatment. Each form that is administered will be a pharmaceutically acceptable form and will optionally comprise a pharmaceutically acceptable carrier.

In one specific embodiment, the method comprises the step of co-administering to a patient in need thereof a) an effective amount of a lactoferrin; and b) an effective amount of an oligomeric proanthocyanidin.

In another specific embodiment, either of the above methods is used to treat a patient suffering from or susceptible to a Pseudomonas aeruginosa infection. A patient susceptible to a Pseudomonas aeruginosa infection specifically includes, but is not limited to, one who is suffering from a disease or condition selected from CF, a burn, a wound, otitis externa, and otitis media.

In an alternate embodiment, either of the above methods is used to treat a patient suffering from or susceptible to a Burkholderia cepacia complex infection. A patient susceptible to a Burkholderia cepacia complex infection is one who is immunocompromised. More specifically, a patient susceptible to a Burkholderia cepacia complex infection includes, but is not limited to, one who is suffering from CF or chronic granulomatous disease.

In still another alternate embodiment, either of the above methods is used to treat a patient suffering from or susceptible to an infection caused by Staphylococcus aureus, methicillin-resistant Staphylococcus aureus (MRSA), Achromobacter xylosoxidans, Hemophilus influenza, or Stenotrophomonas maltophilia. More specifically, a patient suffering or susceptible to an infection caused by Staphylococcus aureus, methicillin-resistant Staphylococcus aureus (MRSA), Achromobacter xylosoxidans, Hemophilus influenza, or Stenotrophomonas maltophilia includes, but is not limited to, one who is suffering from CF. In a more specific embodiment, a patient suffering or susceptible to an infection caused by Staphylococcus aureus, methicillin-resistant Staphylococcus aureus (MRSA), or Hemophilus influenza infection is a pediatric patient suffering from CF. In another specific embodiment, a patient suffering or susceptible to an infection caused by Stenotrophomonas maltophilia is a patient suffering from CF or is a patient who is immunocompromised.

In another embodiment, the patient is suffering from cystic fibrosis and each active ingredient is independently administered in an inhalable composition, an oral composition or a buccal composition.

In another embodiment, the patient is suffering from a gram negative bacterial respiratory infection and each of the active ingredients is co-administered in an inhalable composition.

In a more specific embodiment, the patient is suffering from a gram negative bacterial respiratory infection associated with cystic fibrosis and each of the active ingredients is co-administered in an inhalable composition.

In yet another embodiment, the patient is suffering from otitis externa or otitis media and each of the active ingredients is coadministered to the ear in a topical composition.

In still another embodiment, the invention provides a method of enhancing the bactericidal or bacteriostatic effect of a lactoferrin in a patient in need thereof comprising the step of co-administering to the patient an effective amount of an oligomeric proanthocyanidin; or an effective amount of a protease inhibitor with the lactoferrin. In a specific embodiment, this method comprises the step of co-administering to the patient in need thereof an effective amount of an oligomeric proanthocyanidin with the lactoferrin.

In one specific embodiment, the bactericidal or bacteriostatic effect of a lactoferrin to be enhanced is against bacteria selected from Pseudomonas aeruginosa and a member of the Burkholderia cepacia complex.

Any of the above therapies may additionally comprise co-administering to the patient in need thereof an additional therapeutic agent. For treatment of gram negative bacterial respiratory infection, additional therapeutic agents to be co-administered include, but are not limited to, mucus dissolvers, such as dornase alfa (Pulmozyme®); and antibiotics other than a lactoferrin or an oligomeric proanthocyanidin, such as colistin, tobramycin, azithromycin and ciprofloxacin. For treatment of wounds, additional therapeutic agents to be co-administered include, but are not limited to, a propylene glycol hydrogel (e.g., SOLUGEL® (Johnson & Johnson)); a combination of a glycol, a cellulose derivative and a water soluble aluminum salt, such as described in US 6,958,159; an antiseptic; an antibiotic other than a lactoferrin or an oligomeric proanthocyanidin; and a corticosteroid. For treatment of otitis, additional therapeutic agents to be co-administered include, but are not limited to, antibiotics other than a lactoferrin or an oligomeric proanthocyanidin, such as an azalide antibiotic disclosed in United States Patent Publication 20060046970; steroidal or non-steroidal antiinflammatory agent; antivirals; antifungals; anesthetics; and anti-allergic agents.

The compositions of the invention may also be used in a method for treating a wound comprising at least two of the steps of: a) applying to the wound or to a wound dressing a topical composition comprising a pharmaceutically and dermatologically acceptable carrier and a lactoferrin; b) applying to the wound or to a wound dressing a topical composition comprising a pharmaceutically and dermatologically acceptable carrier and an oligomeric proanthocyanidin; and c) applying to the wound or to a wound dressing a topical composition comprising a pharmaceutically and dermatologically acceptable carrier and a protease inhibitor other than an oligomeric proanthocyanidin. Each of topical compositions a), b) and c) that is utilized in the method may be in a dosage form separate from any other of the compositions, or in a combined dosage form together with one or two the other compositions. When one or more of the compositions is applied to a wound dressing, the method comprises the additional step of placing the wound dressing on the wound.

In one particular embodiment, the method comprises the steps of: a) applying to the wound or to a wound dressing a topical composition comprising a pharmaceutically and dermatologically acceptable carrier and a lactoferrin; and b) applying to the wound or to a wound dressing a topical composition comprising a pharmaceutically and dermatologically acceptable carrier and an oligomeric proanthocyanidin.

In one embodiment, the wound that is treatable by the methods of the present invention is a burn. Such burns include thermal burns, electrical burns or radiation bums.

### Kits

In yet another embodiment, the invention provides a pharmaceutical kit comprising, in separate containers, at least two agents selected from: an oligomeric proanthocyanidin; a protease inhibitor; and a lactoferrin. In a specific embodiment, each agent in the kit is present in a pharmaceutically acceptable carrier.

In certain embodiments of the kits of this invention, the kit additionally comprises another active agent. The choice of other active agent in the kit will depend upon the intended use for the kit. For treatment of gram negative bacterial respiratory infection, additional therapeutic agents that may be present in the kit include, but are not limited to, mucus dissolvers, such as dornase alfa (Pulmozyme®); and antibiotics other than a lactoferrin or an oligomeric proanthocyanidin, such as colistin, tobramycin, azithromycin and ciprofloxacin. For treatment of wounds, additional therapeutic agents that may be present in the kit include, but are not limited to, a propylene glycol hydrogel (e.g., SOLUGEL® (Johnson & Johnson)); a combination of a glycol, a cellulose derivative and a water soluble aluminum salt, such as described in US 6,958,159; an antiseptic; an antibiotic other than a lactoferrin or an oligomeric proanthocyanidin; and a corticosteroid. For treatment of otitis, additional therapeutic agents that may be present in the kit include, but are not limited to, antibiotics other than a lactoferrin or an oligomeric proanthocyanidin, such as an azalide antibiotic disclosed in United States Patent Publication 20060046970; steroidal or non-steroidal antiinflammatory agent; antivirals; antifungals; anesthetics; and anti-allergic agents. The additional therapeutic agent may be present in a container separate from the at least two of an oligomeric proanthocyanidin; a protease inhibitor; and a lactoferrin present in the kit or in the same container as one of those components.

In another embodiment, the kit may additionally comprise a wound dressing which is to be used in conjunction with the therapeutic agents therein.

In certain embodiments, the kit may additionally comprise instructions describing a method of using the components of the kit to treat a disease or condition. Such methods may include mixing the contents of the separate containers prior to administration to a patient.

The container may be any vessel or other sealed or sealable apparatus that can hold said pharmaceutical composition. Examples include bottles, ampules, divided or multi-chambered holders bottles, wherein each division or chamber comprises a single dose of said composition, a divided foil packet wherein each division comprises a single dose of said composition, or a dispenser that dispenses single doses of said composition. The container can be in any conventional shape or form as known in the art which is made of a suitable material, for example a paper or cardboard box, a glass or plastic bottle or jar, a sealed container for use with an inhaler, a re-sealable bag (for example, to hold a "refill" of tablets for placement into a different container), or a blister pack with individual doses for pressing out of the pack according to a therapeutic schedule. The container employed can depend on the exact dosage form involved, for example a conventional cardboard box would not generally be used to hold a liquid suspension. It is feasible that more than one container can be used together in a single package to market a single dosage form. For example, tablets may be contained in a bottle, which is in turn contained within a box.

The kits of this invention may also comprise a device to administer or to measure out a unit dose of one or more of the pharmaceutical composition. Such device may include an inhaler or other appropriate delivery device if said composition is an inhalable composition; a syringe, spoon, pump, or a vessel with or without volume markings if said composition is an oral liquid composition; or any other measuring or delivery device appropriate to the dosage formulation of the composition present in the kit.

### Examples

I. A 41 year-old male cystic fibrosis patient described his asthma as increasingly severe and being present constantly during the day. He reported being more short-breathed than previously, often experiencing difficulty breathing after walking only 50 meters. He also reported an increasingly persistent cough, which escalated to almost every minute. He also described coughing up a "fluorescent light green" mucus. The patient further reported losing about 3.5 kg of weight.

After a lab test by his cystic fibrosis doctor, the patient was diagnosed with a *Pseudomonas aeruginosa* infection. He was prescribed the antibiotics ciprofloxacin (2 X 1000mg), Zithromax® (azithromycin, 1 X 500 mg daily) and TOBI, an aerosolized form of tobramycin (2 X 5mg). The patient reported an initial noticeable decrease in both the asthma and coughing, but after three months on these antibiotics the severity of his asthma, frequency of coughing and quantity of expectorated mucus began increasing.

A second lab test revealed that the *Pseudomonas aeruginosa* infection persisted and that a biofilm of these bacteria had formed. His doctor informed the patient that the biofilm would make the antibiotics less effective and that his disease and lung infections would become worse.

The patient dissolved varying amounts of grape seed powder containing 20% (w/w) of oligomeric proanthocyanidin (OPC) in 37% alcohol/water to produce solutions having OPC concentrations of 1,000 mg/ml, 2,000 mg/ml and 4,000 mg/ml. Each of the solutions were filtered the resulting filtrate then diluted 1:1 with water to produce solutions having final concentrations of 500 mg OPC/1, 1,000 mg OPC/1 and 2,000 mg OPC/1. He loaded 3 ml of the 500 mg OPC/1 solution into an inhaler and self-administered the solution to his lungs two to three times/day for a few days. The solution did not cause his asthma to worsen. He performed the same experiment with each of the higher concentration OPC solutions. No worsening of his asthma occurred.

The patient then prepared a solution of bovine apolactoferrin. The lactoferrin was manufactured by Life Extension as a solid in a capsule. The patient broke open capsules and dissolved the lactoferrin therein in 18.5% alcohol/water to final concentrations ranging from 1 g lactoferrin/1 to 4.5 g lactoferrin/1. Inhaling 3 ml twice to three times per day for a few days of all concentrations of the lactoferrin solution had no immediate deleterious side effects. The patient reported a relief from all signs of his infection, including coughing, for 4 to 5 hours following administration of the lactoferrin. After that time, his condition began to return to the pre-treatment state.

The patient next prepared a solution containing both bovine apolactoferrin and OPCs. He dissolved 900 mg lactoferrin in 50 ml water containing 0.9M NaCl. In a separate container he dissolved 500 mg of grape seed powder (containing 100 mg OPCs) in 50 ml of 37% alcohol/water. The grape seed powder solution was filtered and the filtrate combined with the lactoferrin solution. After 15 minutes the combined solution was again filtered. The resulting filtrate contained 18.5% alcohol, 0.45M NaCl, 900 mg bovine apolactoferrin; and 100 mg OPC per 100ml.

The patient mixed 3 ml of the above solution with 3 ml of 0.9M NaCl and loaded the resulting mixture into an inhaler just before bedtime. The next morning the patient reported no signs of infection and no mucus production upon coughing. This effect lasted for the full 24 hours until the patient's next administration of the OPC/lactoferrin composition - at least 5 times longer than lactoferrin alone. Soon after the beginning of the experiment, the patient ceased taking all of his other daily medications, including pulmozyme and antibiotics. The patient continued this once a day regimen of the OPC/lactoferrin combination for six months without any symptoms or signs of a significant *Pseudomonas* infection. He reported that the volume of mucus expectorated during the course of OPC/lactoferrin treatment was approximately 10% of what it had been prior to beginning that treatment. He also reported an approximate four-fold decrease in coughing frequency while receiving the OPC/lactoferrin combination. The patient's specialist confirmed that the infection was well under control after the end of the six-month experiment. The patient was also easily able to walk to and from a restaurant 750 meters away at the end of the experiment.

Despite the amelioration of the infection, the patient reported more difficulty breathing at the end of the experiment. It is theorized that this may have been due to a buildup in the patient's lungs of contaminants present in the OPC/lactoferrin preparation that could not be removed by filtration prior to administration. Without being bound by theory, applicants believe that the use of more pure lactoferrin and/or OPC sources, as well as the use of human lactoferrin or lactoferrin fragments might increase the efficacy of this combination and/or reduce unwanted side effects. Applicants further believe that the inability of CF patients to produce a sufficiently watery lung secretion due to the high mucus content and the patient's cessation of inhaling vaporized 0.9 N NaCl (which he previously had typically administered 4 to 5 times per day with his other inhalable medications) also reduced his ability to remove the unfilterable contaminants. We believe that administration of mucus dissolvers one or more times daily will also increase the efficacy of this combination and/or reduce unwanted side effects.

The patient ceased using the lactoferrin/OPC combination in an attempt to improve his breathing and resumed taking TOBI twice a day and Zithromax, along with pulmozyme (domase alfa) and additional vaporized 0.9N NaCl. The patient reported that the *Pseudomonas* infection worsened after stopping the lactoferrin/OPC combination, as judged by increased frequency of coughing and larger volume of mucus expectoration, but that his breathing improved. This treatment lasted for approximately four months.

The patient reports that during the past 3 months he has resumed using the lactoferrin/OPC combination, as well as TOBI twice a day and Zithromax, and pulmozyme. He reports that his breathing is clear, and that the *Pseudomonas* infection is once again under control. He further reports greatly reduced use of his asthma inhaler which prior to treatment with the lactoferrin/OPC combination he used 2 to 3 times per day. He also reports less frequency of coughing and lower amounts of mucus being expectorated.

### II. Exemplary test protocol:

1. For a given pathogen, such as a standard laboratory strain of Pseudomonas aeruginosa (PAO1) (e.g., grown in a standard growth medium, such as 10%TSB), determine the minimum inhibitory concentration (MIC) for apo-lactoferrin alone, and the MIC for OPC-containing extract in the presence of sub-MIC level of lactoferrin.
1b. If a MIC is found for the combination of lactoferrin and OPC-containing extract, determine the MIC for OPC-containing extract without lactoferrin.
2. Measure the extent of killing of stationary phase cells by OPC at 10X MIC (or highest tested concentration) and sub-MIC lactoferrin. Also test lactoferrin alone in the absence of OPC.
3. Determine whether OPC can block biofilm development alone, or improve lactoferrin biofilm blocking in a microtiter dish assay. Also test whether lactoferrin in the absence of OPC blocks biofilm development. Determine the stability of lactoferrin during the incubation period of both assays by using SDS-PAGE to quantify full length lactoferrin at several time points.
4. Determine whether sub-MIC concentration of OPC extract affects biofilms growing in a flow cell by scanning confocal microscopy. Test lactoferrin in the absence of OPC in the same assay.

## Claims

1. An inhalable composition comprising
(i) a pharmacetically acceptable carrier and at least two components selected from: an oligomeric proanthocyanidin; a protease inhibitor; and a lactoferrin;
(ii) a pharmaceutically acceptable carrier; at least two components selected from: an oligomeric proanthocyanidin, a protease inhibitor, and a lactoferrin; and an additional therapeutic agent selected from a mucus dissolving agent and an antibiotic other than a lactoferrin or an oligomeric proanthocyanidin; or
(iii) a pharmaceutically acceptable carrier; at least two components selected from: an oligomeric proanthocyanidin, a protease inhibitor, and a lactoferrin, and an additional therapeutic agent selected from domase alfa, colstin, tobramycin, azithromycin and ciprofloxacin.

2. A topical composition comprising:
(i) an oligomeric proanthocyanidin; a protease inhibitor; and a pharmaceutically and dermatologically or otically acceptable carrier; and/or
(ii) an oligomeric proanthocyanidin; a protease inhibitor; a lactoferrin; and a pharmaceutically and dermatologically or otically acceptable carrier; and/or
(iii) a lactoferrin; a protease inhibitor; and a pharmaceutically and dermatologically or otically acceptable carrier; and
(iv) an additional component for use to treat topical bums; and/or
(v) an additional component for use to treat topical burns, wherein the additional component is selected from a propylene glycol hydrogel, a combination of a glycol, a cellulose derivative and a water soluble aluminum salt; an antiseptic; an antibiotic other than a lactoferrin or an oligomeric proanthocyanidin; and a corticosteroid; and/or
(vi) an additional component for use to treat otic infections; and/or
(vii) an additional component for use to treat otic infections, wherein the additional component is selected from an antibiotic other than a lactoferrin or an oligomeric proanthocyanidin; a steroidal antiinflammatory agent; a non-steroidal antiinflammatory agent; an antiviral; an antifungal; an anesthetic; and an anti-allergic agent.

3. A topical composition according to claim 2, wherein the composition is in a form selected from emulsions, creams, lotions, gels, essences, milks, suspensions, or patches.

4. An oral or buccal composition comprising:
(i) a lactoferrin; at least one agent selected from an oligomeric proanthocyanidin and a protease inhibitor; and a pharmaceutically acceptable carrier;
(ii) a lactoferrin; an oligomeric proanthocyanidin; and a pharmaceutically acceptable carrier; or
(iii) a lactoferrin; a protease inhibitor; and a pharmaceutically acceptable carrier.

5. A composition according to any one of claims 1 to 4 which contains a protease inhibitor, wherein
(i) the protease inhibitor inhibits a protease secreted by gram negative bacteria;
(ii) the protease inhibitor inhibits a protease secreted by Pseudomonas aeruginosa;
(iii) the protease inhibitor inhibits one or more proteases selected from bacterial LasA elastase, LasB elastase, alkaline protease, neutrophile elastase, a cathepsin, macrophage elastase, an acid esterase, a collagenase, a tryptase, a chymase, a kinin, a kallikrein, a tumor necrosis factor, a chymotrypsin, a stromelysin, and a matrix metalloprotease;
(iv) the protease inhibitor is selected from an aspartic acid protease inhibitor, a serine protease inhibitor, a cysteine protease inhibitor, and a metalloprotease inhibitor;
(v) the protease inhibitor is a multispecific protease inhibitor;
(vi) the protease inhibitor is a multispecific protease inhibitor selected from α1-antiprotease, α2-macroglobulin and secretory leucocyte protease inhibitor;
(vii) the protease inhibitor is selected from β1-antigellagenase, α2-antiplasmin, serine amyloid A protein, α1-antichymotrypsin, cystatin C, inter-α-trypsin inhibitor, elafin, elastinal, aprotinin, phenylmethyl sulfonyl fluoride, E-64, leupeptin, TIMP-I, TIMP-2, and 1 , 10-phenanthroline;
(vii) said composition comprises two different protease inhibitors, wherein one of the protease inhibitors is a multispecific protease inhibitor;
(viii) the lactoferrin is selected from a native lactoferrin isolated from a mammal, a recombinantly produced lactoferrin, an variant of lactoferrin comprising a modification at the N-terminal glycine, a human lactoferrin comprising a R210K mutation, bovine lactoferrin and an active fragment of lactoferrin;
(ix) the lactoferrin is a fragment selected from human lactoferrin (1-11), human lactoferrin (1-47), bovine lactoferrin (1-51), bovine lactoferrin (17-41), human lactoferrin (1-333), human lactoferrin (345-692) and human lactoferrin (21-31);
(x) the oligomeric proanthocyanidin in the composition is at least 90% composed of catechin and epicatechin monomers;
(xi) at least 90% of the oligomeric proanthocyanidin in the composition is of the B type;
(xii) dimeric, trimeric and tetrameric procyanidins comprise at least 90% by weight of the oligomeric proanthocyanidins present in the composition; and/or
(xiii) said composition comprises an extract obtained from a natural source selected from grape seeds and maritime pine tree bark.

6. A wound dressing comprising a pre-applied composition, wherein the pre-applied composition comprises:
(i) a pharmaceutically and dermatologically acceptable carrier; and at least two components selected from: a) an oligomeric proanthocyanidin; b) a protease inhibitor; and c) a lactoferrin;
(ii) a pharmaceutically and dermatologically acceptable carrier; at least two components selected from: a) an oligomeric proanthocyanidin; b) a protease inhibitor; and c) a lactoferrin; and a hydro gel or hydrogel-forming composition; or
(iii) a pharmaceutically and dermatologically acceptable carrier; at least two components selected from: a) an oligomeric proanthocyanidin; b) a protease inhibitor; and c) a lactoferrin; and an additional component used to treat topical burns, wherein the additional component is optionally selected from a propylene glycol hydrogel; a combination of a glycol, a cellulose derivative and a water soluble aluminum salt; an antiseptic; an antibiotic other than a lactoferrin or an oligomeric proanthocyanidin; and a corticosteroid.

7. A kit comprising at least two agents selected from: an oligomeric proanthocyanidin; a protease inhibitor; and a lactoferrin, wherein at least of two of said agents are in separate containers.

8. A kit according to claim 7, wherein each of said agents is formulated for use in topical administration and wherein the kit additionally comprises a wound dressing.

9. A kit according to claim 7, comprising an additional agent selected from a propylene glycol hydrogel; a combination of a glycol, a cellulose derivative and a water soluble aluminum salt; an antiseptic; an antibiotic other than a lactoferrin or an oligomeric proanthocyanidin; a corticosteroid; a steroidal antiinflammatory agent; a non-steroidal antiinflammatory agent; an antiviral; an antifungal; an anesthetic; an anti-allergic agent' and a mucus dissolving agent.

## Patentansprüche

1. Inhalierbare Zusammensetzung, die Folgendes umfasst:
(i) einen pharmazeutisch annehmbaren Träger und zumindest zwei aus einem oligomeren Proanthocyanidin, einem Proteaseinhibitor und einem Lactoferrin ausgewählte Komponenten;
(ii) einen pharmazeutisch annehmbaren Träger; zumindest zwei aus einem oligomeren Proanthocyanidin, einem Proteaseinhibitor und einem Lactoferrin ausgewählte Komponenten; und ein zusätzliches, aus einem schleimlösenden Mittel und einem Antibiotikum, das kein Lactoferrin und kein oligomeres Proanthocyanidin ist, ausgewähltes therapeutisches Mittel; oder
(iii) einen pharmazeutisch annehmbaren Träger; zumindest zwei aus einem oligomeren Proanthocyanidin, einem Proteaseinhibitor und einem Lactoferrin ausgewählte Komponenten; und ein zusätzliches, aus Domase α, Colstin, Tobramycin, Azithromyin und Ciprofloxacin ausgewähltes therapeutisches Mittel.

2. Topische Zusammensetzung, die Folgendes umfasst:
(i) ein oligomeres Proanthocyanidin, einen Proteaseinhibitor und einen pharmazeutisch und dermatologisch oder für das Ohr annehmbaren Träger; und/oder
(ii) ein oligomeres Proanthocyanidin, einen Proteaseinhibitor, ein Lactoferrin und einen pharmazeutisch und dermatologisch oder für das Ohr annehmbaren Träger; und/oder
(iii) ein Lactoferrin, einen Proteaseinhibitor und einen pharmazeutisch und dermatologisch oder für das Ohr annehmbaren Träger; und
(iv) eine zusätzliche Komponente zur Verwendung bei der Behandlung topischer Verbrennungen; und/oder
(v) eine zusätzliche Komponente zur Verwendung bei der Behandlung topischer Verbrennungen, worin die zusätzliche Komponente aus einem Propylenglykolhydrogel, einer Kombination aus einem Glykol, einem Cellulosederivat und einem wasserlöslichen Aluminiumsalz; einem Antiseptikum; einem Antibiotikum, das kein Lactoferrin und kein oligomeres Proanthocyanidin ist; und einem Corticosteroid ausgewählt ist; und/oder
(vi) eine zusätzliche Komponente zur Verwendung bei der Behandlung von Ohreninfektionen; und/oder
(vii) eine zusätzliche Komponente zur Verwendung bei der Behandlung von Ohreninfektionen, worin die zusätzliche Komponente aus einem Antibiotikum, das kein Lactoferrin und kein oligomeres Proanthocyanidin ist; einem steroidalen entzündungshemmenden Mittel; einem nicht steroidalen entzündungshemmenden Mittel; einem Virostatikum; einem Antimykotikum; einem Betäubungsmittel; und einem Antiallergikum ausgewählt ist.

3. Topische Zusammensetzung nach Anspruch 2, worin die Zusammensetzung in einer aus Emulsionen, Cremen, Lotionen, Gelen, Essenzen, Milchen, Suspensionen oder Pflastern ausgewählten Form vorliegt.

4. Orale oder bukkale Zusammensetzung, die Folgendes umfasst:
(i) ein Lactoferrin, zumindest ein aus einem oligomeren Proanthocyanidin und einem Proteasinhibitor ausgewähltes Mittel und einen pharmazeutisch annehmbaren Träger;
(ii) ein Lactoferrin, ein oligomeres Proanthocyanidin und einen pharmazeutisch annehmbaren Träger; oder
(iii) ein Lactoferrin, einen Proteaseinhibitor und einen pharmazeutisch annehmbaren Träger.

5. Zusammensetzung nach einem der Ansprüche 1 bis 4, die einen Proteaseinhibitor enthält, worin
(i) der Proteaseinhibitor eine von gramnegativen Bakterien sezernierte Protease hemmt;
(ii) der Proteaseinhibitor eine von *Pseudomonas aeruginosa* sezernierte Protease hemmt;
(iii) der Proteaseinhibitor eine oder mehrere aus bakterieller LasA-Elastase, LasB-Elastase, alkalischer Protease, Neutrophilelastase, einem Cathepsin, Makrophagenelastase, einer Säureesterase, einer Kollagenase, einer Tryptase, einer Chymase, einem Kinin, einem Kallikrein, einem Tumornekrosefaktor, einem Chymotrypsin, einem Stromelysin und einer Matrixmetalloprotease ausgewählte Proteasen hemmt;
(iv) der Proteaseinhibitor aus einem Asparaginsäureproteaseinhibitor, einem Serinproteaseinhibitor, einem Cysteinproteaseinhibitor und einem Metalloproteaseinhibitor ausgewählt ist;
(v) der Proteaseinhibitor ein multispezifischer Proteaseinhibitor ist;
(vi) der Proteaseinhibitor ein aus α1-Antiprotease, α2-Makroglobulin und sekretorischem Leukozytenproteaseinhibitor ausgewählter multispezifischer Proteaseinhibitor ist;
(vii) der Proteaseinhibitor aus β1-Antigellagenase, α2-Antiplasmin, Serin-Amyloid-A-Protein, α1-Antichymotrypsin, Cystatin C, Inter-α-Trypsin-Inhibitor, Elafin, Elastinal, Aprotinin, Phenylmethylsulfonylfluorid, E-64, Leupeptin, TIMP-1, TIMP-2 und 1,10-Phenanthrolin ausgewählt ist;
(vii) die Zusammensetzung zwei verschiedene Proteaseinhibitoren umfasst, worin einer der Proteaseinhibitoren ein multispezifischer Proteaseinhibitor ist;
(viii) das Lactoferrin aus einem von einem Säugetier isolierten nativen Lactoferrin, einem rekombinant hergestellten Lactoferrin, einer Variante von Lactoferrin, die eine Modifikation am N-terminalen Glycin umfasst, einem menschlichen Lactoferrin, das eine Mutation R210K umfasst, Rinder-Lactoferrin und einem aktiven Fragment von Lactoferrin ausgewählt ist;
(ix) das Lactoferrin ein aus menschlichem Lactoferrin-(1-11), menschlichem Lactoferrin-(1-47), Rinder-Lactoferrin-(1-51), Rinder-Lactoferrin-(17-41), menschlichem Lactoferrin-(1-333), menschlichem Lactoferrin-(345-692) und menschlichem Lactoferrin-(21-31) ausgewähltes Fragment ist;
(x) das oligomere Proanthocyanidin in der Zusammensetzung zu zumindest 90 % aus Catechin- und Epicatechinmonomeren besteht;
(xi) zumindest 90 % des oligomeren Proanthocyanidins in der Zusammensetzung dem Typ B angehören;
(xii) dimere, trimere und oligomere Proanthocyanidine zumindest 90 Gew.-% des in der Zusammensetzung vorhandenen oligomeren Proanthocyanidins ausmachen; und/oder
(xiii) die Zusammensetzung einen aus einer aus Traubenkernen und Seekiefernrinde ausgewählten natürlichen Quelle gewonnenen Extrakt umfasst.

6. Wundauflage, die eine vorab aufgetragene Zusammensetzung umfasst, worin die vorab aufgetragene Zusammensetzung Folgendes umfasst:
(i) einen pharmazeutisch und dermatologisch annehmbaren Träger und zumindest zwei aus a) einem oligomeren Proanthocyanidin, b) einem Proteaseinhibitor und c) einem Lactoferrin ausgewählte Komponenten;
(ii) einen pharmazeutisch annehmbaren Träger; zumindest zwei aus a) einem oligomeren Proanthocyanidin, b) einem Proteaseinhibitor und c) einem Lactoferrin ausgewählte Komponenten; und ein Hydrogel oder eine hydrogelbildende Zusammensetzung; oder
(iii) einen pharmazeutisch und dermatologisch annehmbaren Träger, zumindest zwei aus a) einem oligomeren Proanthocyanidin, b) einem Proteaseinhibitor und c) einem Lactoferrin ausgewählte Komponenten; und eine zusätzliche Komponente zur Verwendung bei der Behandlung topischer Verbrennungen, worin die zusätzliche Komponente gegebenenfalls aus einem Propylenglykolhydrogel, einer Kombination aus einem Glykol, einem Cellulosederivat und einem wasserlöslichen Aluminiumsalz; einem Antiseptikum; einem Antibiotikum, das kein Lactoferrin und kein oligomeres Proanthocyanidin ist; und einem Corticosteroid ausgewählt ist.

7. Set, das zumindest zwei aus einem oligomeren Proanthocyanidin, einem Proteaseinhibitor und einem Lactoferrin ausgewählte Mittel umfasst, worin zumindest zwei der Mittel in gesonderten Behältern vorliegen.

8. Set nach Anspruch 7, worin jedes der Mittel zur Verwendung bei der topischen Verabreichung formuliert ist und worin das Set außerdem eine Wundauflage umfasst.

9. Set nach Anspruch 7, das ein zusätzliches, aus einem Propylenglykolhydrogel, einer Kombination aus einem Glykol, einem Cellulosederivat und einem wasserlöslichen Aluminiumsalz; einem Antiseptikum; einem Antibiotikum, das kein Lactoferrin und kein oligomeres Proanthocyanidin ist; einem Corticosteroid; einem steroidalen entzündungshemmenden Mittel; einem nicht steroidalen entzündungshemmenden Mittel; einem Virostatikum; einem Antimykotikum; einem Betäubungsmittel; einem Antiallergikum; und einem schleimlösenden Mittel ausgewähltes Mittel umfasst.

## Revendications

1. Composition inhalable comprenant .
(i) un véhicule pharmaceutiquement acceptable et au moins deux composants choisis parmi : une proanthocyanidine oligomère ; un inhibiteur de protéase ; et une lactoferrine ;
(ii) un véhicule pharmaceutiquement acceptable ; au moins deux composants choisis parmi : une proanthocyanidine oligomère, un inhibiteur de protéase, et une lactoferrine ; et un agent thérapeutique additionnel choisi parmi un agent dissolvant le mucus et un antibiotique autre qu'une lactoferrine ou une pro-anthocyanidine oligomère ; ou
(iii) un véhicule pharmaceutiquement acceptable ; au moins deux composants choisis parmi : une proanthocyanidine oligomère, un inhibiteur de protéase, et une lactoferrine ; et un agent thérapeutique additionnel choisi parmi la domase alfa, la colstine, la tobramycine, l'azithromycine et la ciprofloxacine.

2. Composition à usage topique comprenant :
(i) une pro-anthocyanidine oligomère ; un inhibiteur de protéase ; et un véhicule acceptable des points de vue pharmaceutique et dermatologique ou otique ; et/ou
(ii) une pro-anthocyanidine oligomère ; un inhibiteur de protéase ; une lactoferrine ; et un véhicule acceptable des points de vue pharmaceutique et dermatologique ou otique ; et/ou
(iii) une lactoferrine ; un inhibiteur de protéase ; et un véhicule acceptable des points de vue pharmaceutique et dermatologique ou otique ; et
(iv) un composant additionnel pour utilisation dans le traitement de brûlures locales ; et/ou
(v) un composant additionnel pour utilisation dans le traitement de brûlures locales, le composant additionnel étant choisi parmi un hydrogel de propylèneglycol, une combinaison d'un glycol, d'un dérivé de cellulose et d'un sel d'aluminium soluble dans l'eau ; un antiseptique ; un antibiotique autre qu'une lactoferrine ou une proanthocyanidine oligomère ; et un corticostéroïde ; et/ou
(vi) un composant additionnel pour utilisation dans le traitement d'infections otiques ; et/ou
(vii) un composant additionnel pour utilisation dans le traitement d'infections otiques, le composant additionnel étant choisi parmi un antibiotique autre qu'une lactoferrine ou une pro-anthocyanidine oligomère ; un agent anti-inflammatoire stéroïdien ; un agent anti-inflammatoire non stéroïdien ; un antiviral ; un antifongique ; un anesthésique ; et un agent antiallergique.

3. Composition à usage topique selon la revendication 2, laquelle composition est sous une forme choisie parmi les émulsions, les crèmes, les lotions, les gels, les essences, les laits, les suspensions, et les timbres.

4. Composition à usage oral ou buccal comprenant :
(i) une lactoferrine ; au moins un agent choisi parmi une pro-anthocyanidine oligomère et un inhibiteur de protéase ; et un véhicule pharmaceutiquement acceptable ;
(ii) une lactoferrine ; une pro-anthocyanidine oligomère ; et un véhicule pharmaceutiquement acceptable ; ou
(iii) une lactoferrine ; un inhibiteur de protéase ; et un véhicule pharmaceutiquement acceptable.

5. Composition selon l'une quelconque des revendications 1 à 4 qui contient un inhibiteur de protéase, dans laquelle
(i) l'inhibiteur de protéase inhibe une protéase sécrétée par des bactéries Gram négatives ;
(ii) l'inhibiteur de protéase inhibe une protéase sécrétée par Pseudomonas aeruginosa ;
(iii) l'inhibiteur de protéase inhibe une ou plusieurs protéases choisies parmi l'élastase LasA bactérienne, l'élastase LasB, la protéase alcaline, l'élastase de neutrophiles, une cathepsine, l'élastase de macrophages, une estérase acide, une collagénase, une tryptase, une chymase, une kinine, une kallikréine, un facteur nécrosant les tumeurs, une chymotrypsine, une stromélysine, et une métalloprotéase de matrice ;
(iv) l'inhibiteur de protéase est choisi parmi un inhibiteur d'acide aspartique protéase, un inhibiteur de sérine protéase, un inhibiteur de cystéine protéase, et un inhibiteur de métalloprotéase ;
(v) l'inhibiteur de protéase est un inhibiteur de protéase multispécifique ;
(vi) l'inhibiteur de protéase est un inhibiteur de protéase multispécifique choisi parmi l'α1-antiprotéase, l'α2-macroglobuline et l'inhibiteur de protéase leucocytaire sécrétoire ;
(vii) l'inhibiteur de protéase est choisi parmi la β1-antigellagénase, l'α2-antiplasmine, la protéine A de sérine amyloïde, l'α1-antichymotrypsine, la cystatine C, l'inhibiteur d'inter-α-trypsine, l'élafine, l'élastinal, l'aprotinine, le fluorure de phénylméthylsulfonyle, E-64, la leupeptine, TIMP-1, TIMP-2, et la 1,10-phénanthroline ;
(vii) ladite composition comprend deux inhibiteurs de protéase différents, l'un des inhibiteurs de protéase étant un inhibiteur de protéase multispécifique ;
(viii) la lactoferrine est choisie parmi une lactoferrine native isolée à partir d'un mammifère, une lactoferrine produite par recombinaison, un variant de lactoferrine comprenant une modification au niveau de la glycine N-terminale, une lactoferrine humaine comprenant une mutation R210K, la lactoferrine bovine et un fragment actif de lactoferrine ;
(ix) la lactoferrine est un fragment choisi parmi les fragments de lactoferrine humaine (1-11), de lactoferrine humaine (1-47), de lactoferrine bovine (1-51), de lactoferrine bovine (17-41), de lactoferrine humaine (1-333), de lactoferrine humaine (345-692), et de lactoferrine humaine (21-31) ;
(x) la pro-anthocyanidine oligomère dans la composition est composée à au moins 90 % de monomères de catéchine et d'épicatéchine ;
(xi) au moins 90 % de la pro-anthocyanidine oligomère dans la composition sont de type B ;
(xii) les procyanidines dimères, trimères et tétramères représentent au moins 90 % en poids des proanthocyanidines oligomères présentes dans la composition ; et/ou
(xiii) ladite composition comprend un extrait obtenu à partir d'une source naturelle choisie parmi les pépins de raisin et l'écorce de pin maritime.

6. Pansement comprenant une composition préappliquée, dans lequel la composition pré-appliquée comprend :
(i) un véhicule acceptable des points de vue pharmaceutique et dermatologique ; et au moins deux composants choisis parmi : a) une pro-anthocyanidine oligomère ; b) un inhibiteur de protéase ; et c) une lactoferrine ;
(ii) un véhicule acceptable des points de vue pharmaceutique et dermatologique ; au moins deux composants choisis parmi : a) une pro-anthocyanidine oligomère ; b) un inhibiteur de protéase ; et c) une lactoferrine ; et un hydrogel ou une composition formant un hydrogel ; ou
(iii) un véhicule acceptable des points de vue pharmaceutique et dermatologique ; au moins deux composants choisis parmi : a) une pro-anthocyanidine oligomère ; b) un inhibiteur de protéase ; et c) un composant additionnel utilisé pour traiter des brûlures locales, le composant additionnel étant éventuellement choisi parmi un hydrogel de propylèneglycol ; une combinaison d'un glycol, d'un dérivé de cellulose et d'un sel d'aluminium soluble dans l'eau ; un antiseptique ; un antibiotique autre qu'une lactoferrine ou une pro-anthocyanidine oligomère ; et un corticostéroïde.

7. Trousse comprenant au moins deux agents choisis parmi : une pro-anthocyanidine oligomère ; un inhibiteur de protéase ; et une lactoferrine, dans laquelle au moins deux desdits agents sont dans des récipients séparés.

8. Trousse selon la revendication 7, dans laquelle chacun desdits agents est formulé pour une utilisation dans une administration topique, laquelle trousse comprend de plus un pansement.

9. Trousse selon la revendication 7, comprenant un agent additionnel choisi parmi un hydrogel de propylèneglycol ; une combinaison d'un glycol, d'un dérivé de cellulose et d'un sel d'aluminium soluble dans l'eau ; un antiseptique ; un antibiotique autre qu'une lactoferrine ou une pro-anthocyanidine oligomère ; un corticostéroïde ; un agent anti-inflammatoire stéroïdien ; un agent antiinflammatoire non stéroïdien ; un antiviral ; un antifongique ; un anesthésique ; et un agent antiallergique et un agent dissolvant le mucus.
